# EUROPEAN PATENT SPECIFICATION

(11) **EP 0 391 452 B1**
(45) Date of publication and mention of the grant of the patent: **05.01.1994**
(21) Application number: 90109462.3
(22) Date of filing: 23.08.1984
(51) Int. Cl.: A61F 2/16

(54) **Intraocular lens implants**
Intraokulares Linsenimplantat
Implant de lentille intraoculaire

(30) Priority: 30.08.1983 AU 1120/83
(43) Date of publication of application: 10.10.1990
(62) Divisional of application: 84305762.1
(73) Proprietor: EZEKIEL NOMINEES PTY.LTD., West Perth Western Australia (AU)
(72) Inventor: Barrett, Graham David, City Beach, Western Australia (AU)
(74) Representative: Bond, Bentley George

(56) References cited:
- US-A- 3 961 379
- US-A- 4 242 762
- US-A- 4 249 272
- US-A- 4 254 509
- US-A- 4 449 257
- SYNTHETIC BIOMEDICAL POLYMERS CONCEPTS AND APPLICATIONS, 1982, pages 171-185,Technomic Publishing Co., Westport, US; M.F. REFOJO: "Ophthalmic hydrogels"

## Description

The present invention relates to intraocular lens.

It is known to replace the natural crystalline lens following cataract extraction. Many people on whom cataract operations are performed receive an intraocular lens implant. However, there is a need for a safer and more effective intraocular lens than those which have been available hitherto. The most common used material for intraocular lens has been polymethylmethacrylate (PMMA). PMMA has a number of characteristics making it suitable for use as an intraocular lens implant. However, it has been shown to be particularly injurious to the corneal endothelium. The corneal endothelium is a thin layer at the back of the cornea. The maintenance of corneal clarity is dependent on the endothelium which is essentially non-regenerative. There appears to be a bio-physical interaction between the hydrophobic PMMA and the endothelium such that even momentary touch on insertion will cause significant endothelial cell disruption by adherence of the cells to the lens surface.

Loss of endothelial cells at the time of surgery can lead to loss of corneal transparency several years later. There are other problems attendant with the use of PMMA as an implant material.

In US-A-4254509, there is disclosed an intraocular lens construction which comprises an eye implant including a dumbell shaped optical lens having an anterior convex surface and a posterior planar surface, and which is supported on diametrically opposed coplanar feet through two supporting members which form an arch. This lens construction can be formed partially or totally of soft materials such as soft hydrogels of hydrophilic materials such as 2-hydroxyethyl methacrylate.

In Am. Intra-Ocular Implant Soc. J. 4, 200-205 (1978), there is disclosed a soft lens implant having a thin front plate which is slightly concave anteriorly to permit easy iris insertion, the back of the lens being lenticularized to decrease its weight.

In US-A-4242762, there is disclosed an artificial intraocular lens for incapsulation in the posterior chamber of an eye, which lens element includes a generally triangular base element with an optic element positioned therein, and a pin extending outwardly from one corner thereof.

In EP-A-0064770, there is disclosed an implantation lens consisting of a central body and of support mountings made of silicone rubber, and having all the features of the preamble of claim 1.

According to the present invention, there is provided a self-supporting intraocular lens suitable for implantation in the posterior chamber of the human eye to replace the natural crystalline lens, and comprising:
an optical portion having an anterior surface and a posterior surface, said optical portion being sufficiently thick and rigid to provide stable optical correction; and
flange means having an anterior surface and a posterior surface, said flange means being resilient and functioning to support and retain the lens in place in the eye following implantation without fixation to the iris of the eye;
said lens being a posterior chamber lens, said flange means projecting anteriorly with respect to the optical portion in the posterior chamber of the eye, and the optical portion being of asymmetrical biconvex construction;
characterised in that said lens is formed entirely of a hydrogel and maintains its shape when in hydrated form and in that the posterior surface of the optical portion has a larger radius of curvature than the anterior surface of the optical portion.

A hydrogel is an organic polymeric or copolymeric material comprising hydrophilic monomers. The hydrogel material swells upon being hydrated and becomes soft and flexible. One particularly useful hydrogel is hydroxyethyl methacrylate (HEMA) and it has been found that this material causes little endothelial damage on contact. Also, since hydrogels are hydrophilic in nature endothelial damage is generally less than with PMMA.

Other types of hydrogel which may be used in the present invention are copolymers of vinyl pyrrolidone with HEMA or methyl methacrylate, copolymers of glyceryl methacrylate and methyl methacrylate and copolymers of HEMA and diacetone acrylamide.

It has been found in particular that a HEMA hydrogel lens manufactured from HEMA having the capability of absorbing about 38% of its weight of water, makes a particularly useful posterior chamber intraocular lens.

Preferably, the intraocular lens implant is of integral construction and comprises a relatively thick optical portion having relatively thin resilient flange means extending away from it, said flange means being arranged to retain the implant in place in the eye.

The present invention will now be described, by way of example, with reference to the accompanying drawings, in which:-
Figure 1 is a plan view of an intraocular lens implant in accordance with one embodiment of the present invention;
Figure 2 is a side elevation of the intraocular lens implant of Figure 1;
Figure 3 is a plan view of an intraocular lens implant in accordance with another embodiment of the present invention;
Figure 4a is a cross-sectional view of an eye with an implant according to Figures 1 and 2 in place with a ciliary sulcus placement;
Figure 4b is a cross-sectional view of an eye with an implant according to Figures 1 and 2 in place with a capsule bag placement;
Figures 5 and 6 are views similar to that of Figure 4a showing alternative intraocular lens configurations in accordance with the present invention;
Figure 7 is a plan view of the lens shown in Figure 6 ; and
Figure 8 is a side elevation of a package containing an intraocular lens implant in accordance with the present invention.

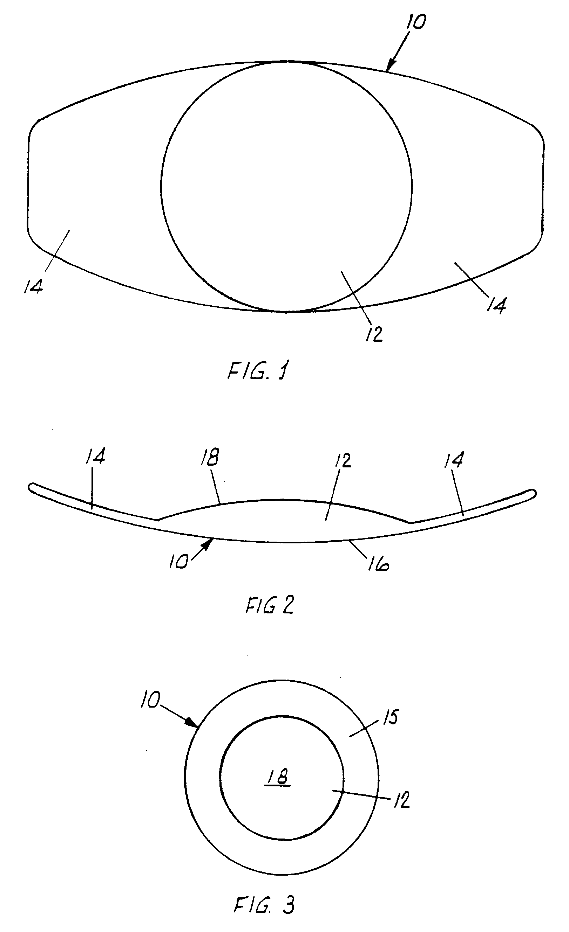

In Figures 1 and 2, there is shown an intraocular lens implant 10 comprising a central optical portion 12 which is in the form of a lens. The central optical portion 12 is flanked by laterally extending flanges 14. The implant has a posterior face 16 and an anterior face 18. The lens implant of Figures 1 and 2 is arranged to be inserted in the posterior chamber of an eye.

As can be seen in Figure 2, the optical portion 12 is of asymmetrical biconvex construction which gives good optical resolution. The posterior face 16 is at a standard curvature such as a curve having a radius of about 17mm. The posterior face 16 is, in this case, a non-variable optical surface whilst the anterior face 18 is an optical surface of varying power. In other lens constructions, the anterior face 18 may be a non-variable optical surface whilst the posterior face 16 may be an optical surface of varying power. Thus, in the embodiment illustrated in Figure 2, the optical properties of the optical portion 12 can be varied by varying the curvature of the anterior face of the optical portion 12. The ratio of curvature is preferably approximately 3:1 which computer analysis shows to provide optimum ocular resolution for an intraocular lens.

The power of each eye is different and therefore the thickness of the optical portion 12 and the curvature of the anterior face 18 thereof will vary from case to case. The technique for forming the correct shape of the anterior face and thickness of the optical portion 12 are known.

The lens implant shown in Figure 3 is similar to that shown in Figures 1 and 2, except that the flanges 14 have been replaced by a single circular flange 15 which completely surrounds the optical portion 12. The flange 15 is of similar cross-sectional shape and thickness to the flanges 14. The curvature of the posterior face 16 is preferably such that the resultant lens will be of asymmetrical biconvex construction with the posterior face having the larger curvature as shown in Figure 2.

Further, the optical portion 12 and flanges 14 are formed in an integral unit, that is the entire implant 10 is formed in one piece. The flanges 14 may be of a wide variety of thickness but are preferably between 0.02 and 0.2mm thick. More preferably, the flanges 14 are between 0.10 and 0.18mm thick such as about 0.14mm thick. The optical portion 12 is thicker than the flanges 14 but, as described above, its actual thickness will vary with optical requirements of the lens implant 10. A typical thickness for the optical portion 12 is about 0.9mm.

The implant 10 is formed of a hydrogel material such as HEMA and the flanges 14 are therefore resilient. However, the optical portion 12 is thick enough to be sufficiently rigid to provide stable optical correction.

As can be seen in Figure 2, the flanges 14 have a similar curvature to the posterior face 16. Thus the flanges 14 project forwardly and, as will be described, dispose the implant 10 away from the iris in use.

Further, the flanges 14 may be transversely tapered as can be seen in Figure 1. This enables the flanges 14 to be inserted even into a small pupil. The flanges 14 may, for example, taper from 6mm at the optical zone 12 to 2mm at their outer extremities.

The lens implant 10 can be manufactured by any suitable technique such as by forming a blank on a lathe, polishing the lens implant, checking the thicknesses of the various parts of the lens implant, checking in the dry state for any flaws, cleaning to remove residual wax or polish and then bathing the implant in saline solution. The hydrated implant can then be washed in a Soxhlet system and again examined for defects in the hydrated state. Other suitable manufacturing techniques include moulding or pressing to form a lens implant in accordance with the present invention. The power of the lens is measured in the hydrated state. The lens dimensions are thus measured in the hydrated state. Finally, the lens implant is placed in a sealed vial in a physiologically acceptable electrolyte solution and autoclaved to sterilise it. The electrolyte solution must be a balanced or isotonic salt solution which will hydrate the lens implant 10 and be compatible with the human eye.

The vial is preferably a glass vial.

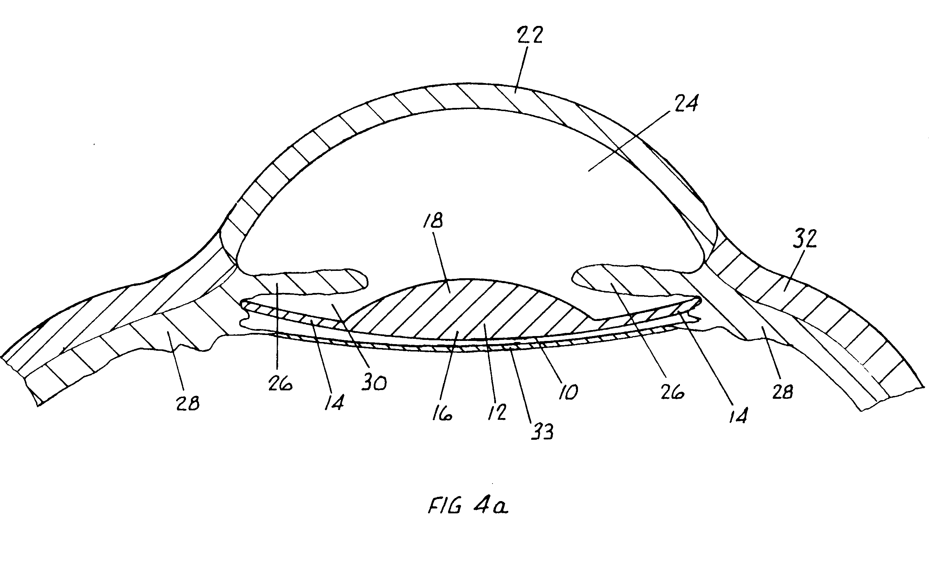

In Figure 4a there is shown an eye comprising a cornea 22 which has an endothelium layer on its inner face. Behind the cornea there is an anterior chamber 24 which is filled with aqueous fluid. At the rear of the chamber 24 there is located the iris 26 which is in two parts separated by a gap which constitutes the pupil of the eye. At its outer edge the iris 26 is connected to ciliary sulcus 28. The region, behind the iris 26 forms a posterior chamber 30 which also contains aqueous fluid. To the front of the ciliary sulcus 28 is the white 32 of the eye. To the rear of the posterior chamber is the posterior capsule 33 of the eye.

As can be seen in Figure 4a the lens implant 10 of Figures 1 and 2 is mounted in the eye in the posterior chamber 30. The lens implant 10 is retained in place by engagement of the flanges 14 in the ciliary sulcus 28. There are two preferred methods of fixation for a posterior chamber lens in accordance with the present invention. The first method is illustrated in Figure 4a in which the lens is fixed in place by engagement with the ciliary sulcus 28 and in this case the lens width may be from about 12 to 14mm such as about 12.5mm.

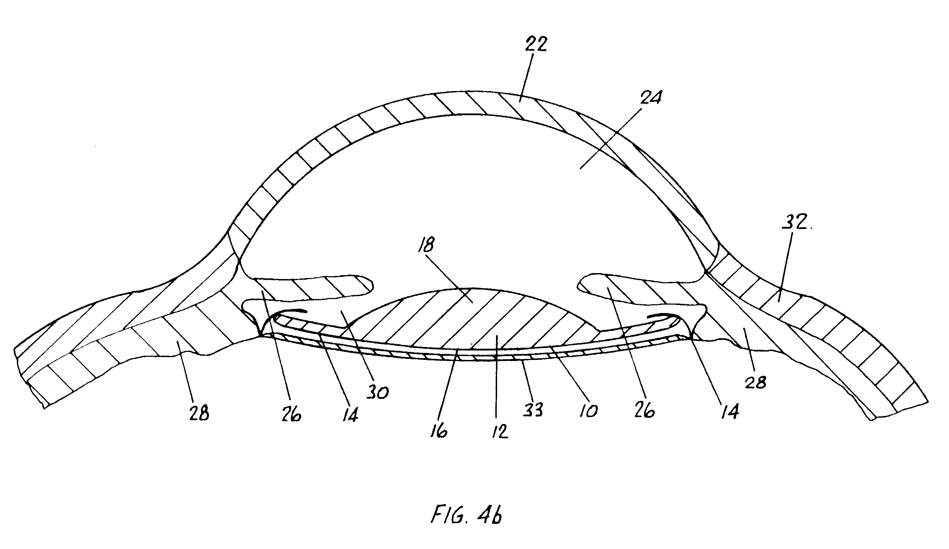

The second method of fixation is illustrated in Figure 4b and is by means of the capsular bag of the eye. In this case the capsular bag, fixes the lens in place. The lens intended for capsular bag placement would typically have a diameter in the range from about 10 to 12mm such as about 11mm. In other respects the lens of Figure 4b is similar to that of Figure 4a.

The intraocular lens implant of the present invention can take many forms.

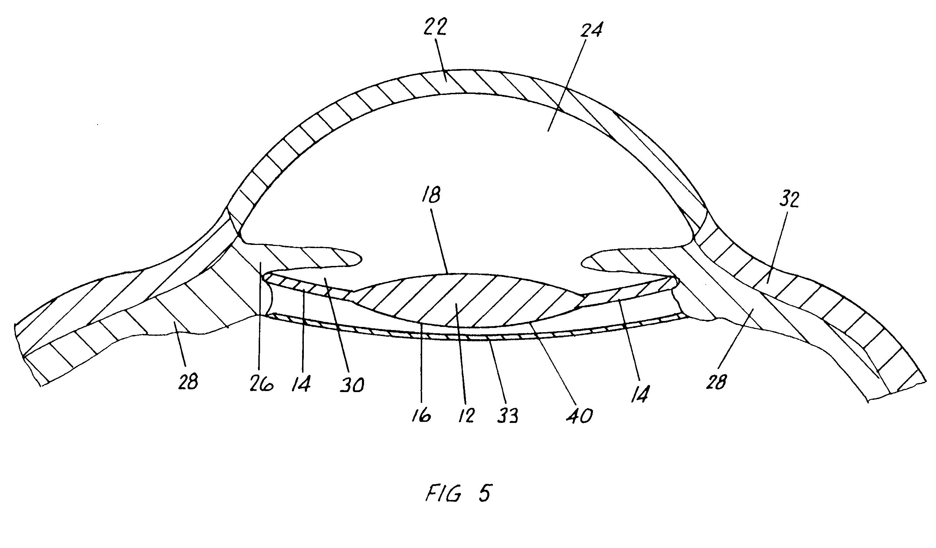

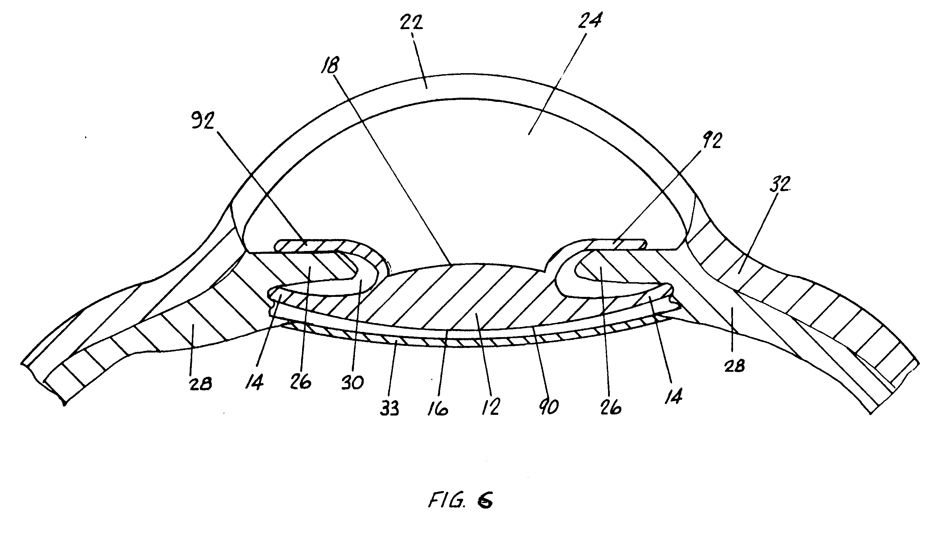

Figures 5 to 7 illustrate various modifications of the lens implant shown in Figure 4 and like reference numerals denote like parts. The lens implants of Figures 5 and 6 are intended for posterior chamber placement by engagement with the ciliary sulcus but equivalent lens can be made which are intended for capsule bag placement as shown in Figure 4b.

An alternative form of intraocular lens implant 40 according to the present invention intended for implantation in the posterior chamber of the eye is shown in Figure 5. In this case, the posterior face 16 is not of uniform curvature throughout but has increased curvature in the optical portion 12. The lens 40 is of asymmetrical biconvex construction.

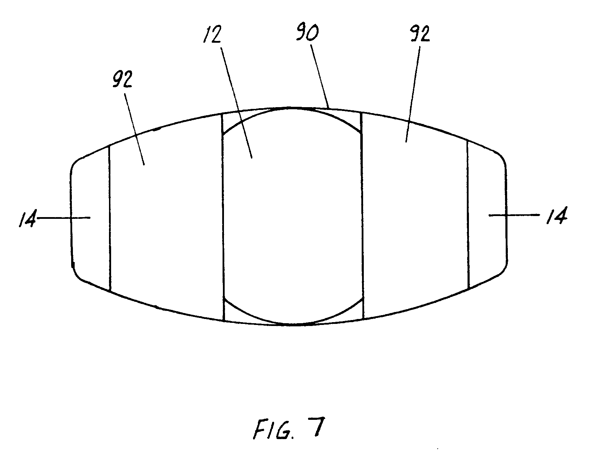

An alternative form of intraocular lens implant 90 according to the present invention intended for implantation in the posterior chamber of the eye is shown in Figures 6 and 7. The lens of Figures 6 and 7 comprise a pair of lugs 92 which extend upwardly and then outwardly from the anterior face 18. As shown in Figure 6, the lugs 92 are each intended to engage with iris 26 of an eye. Thus the lugs 92 hold the lens implant 90 in place by engagement with the iris 26 but not necessarily by engagement with the ciliary sulcus 28. In other respects the lens implant 90 is similar to the lens implant shown in Figure 4.

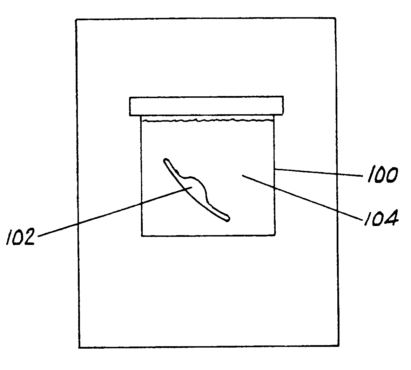

Preferably, a manufactured lens is washed a number of times in double distilled water to remove impurities from it and then autoclaved as described above. The autoclaving may be conducted for 15 to 30 minutes at a pressure in the range from about 120 to 130mm mercury in a sealed vial containing a physiologically acceptable electrolyte solution. Then the sterilised vial is placed inside an internally sterile overpouch which is sealed and the autoclaving process is then repeated to ensure complete sterility of the completed product. A typical package comprising a sealed vial 100 containing a lens implant 102 and a quantity of physiologically acceptable saline solution 104 all contained within a flexible overpouch 106 is shown in Figure 8.

The diameter of the optical portion 12 of a lens implant in accordance with the present invention is preferably from 3 to 10mm, more preferably from 4 to 7mm. The overall length of the lens implant may be from 8 to 15mm.

The non-variable optical surface of the optical portion 12 preferably ranges from plano to 10mm in radius of curvature, preferably from 15 to 30mm radius of curvature. As stated above the radius of curvature of the optical surface of varying power is varied to adjust the optical power of the lens implant.

The lens implant of the present invention is particularly envisaged for use where a cataract has been removed. However, the lens implant of the present invention may be used to correct refractive errors and myopia without prior cataract extraction.

The lens implant of the present invention may include location members such as indentations, recesses or holes to assist in positioning the lens in the middle of the eye. The lens implant can be inserted at the time of cataract extraction or as a secondary implant. The lens can be inserted by the standard procedure. The design of the lens also allows the flanges 14 to be inserted in a folded condition and then be allowed to open out through their own inherent resilience to engage with the ciliary sulcus 28. The flanges 14 avoid the use of prolene hooks or the like which have been used in the past.

Whilst it is preferred to insert the lens in hydrated condition from a vial as shown in Figure 8, the lens implant could be inserted into the eye dry and hydrated subsequently to hydrate and swell it. The advantage of dry insertion is that it allows the lens implant to be inserted through a small wound in the eye.

The lens implant of the present invention may have a built in U.V. filter which is incorporated in the hydrogel. The U.V. filter can be incorporated in the chemical mix as polymerisation takes place or a.U.V. absorbing function can be built into the polymeric chain.

Modifications and variations such as would be apparent to a skilled addressee are deemed within the scope of the present invention.

## Claims

1. A self-supporting intraocular lens (10) suitable for implantation in the posterior chamber (30) of the human eye to replace the natural crystalline lens, and comprising:
an optical portion (12) having an anterior surface (18) and a posterior surface (16), said optical portion (12) being sufficiently thick and rigid to provide stable optical correction; and
flange means (14) having an anterior surface and a posterior surface, said flange means (14) being resilient and functioning to support and retain the lens (10) in place in the eye following implantation without fixation to the iris (26) of the eye;
said lens (10) being a posterior chamber lens, said flange means (14) projecting anteriorly with respect to the optical portion (12) in the posterior chamber (30) of the eye, and the optical portion (12) being of asymmetrical biconvex construction;
characterised in that said lens (10) is formed entirely of a hydrogel and maintains its shape when in hydrated form and in that the posterior surface (16) of the optical portion (12) has a larger radius of curvature than the anterior surface (18) of the optical portion (12).

2. An intraocular lens (10) according to claim 1, wherein said flange means (14) projects anteriorly with respect to the optical portion (12).

3. An intraocular lens (10) according to claim 1, wherein said flange means (14) comprises a pair of flanges extending laterally from opposite sides of the optical portion (12).

4. An intraocular lens (10) according to claim 3, wherein the flanges are imperforate.

## Patentansprüche

1. Selbsttragendes Augenlinsenimplantat (10), das beim Menschen als Ersatz für die natürliche Kristallinse in die hintere Augenkammer (30) einsetzbar ist, umfassend:
ein optisches Teil (12) mit einer vorderen Fläche (18) und einer hinteren Fläche (16), wobei das optische Teil (12) hinreichend dick und steif ist, um eine stabile optische Korrektur zu gewährleisten; und
eine Flanscheinrichtung (14) mit einer vorderen und einer hinteren Fläche, die nachgiebig ist und dazu dient, nach der Implantation die Linse (10) ohne eine Befestigung an der Augen-Iris (26) in Stellung zu halten;
wobei die Linse (10) eine Hinterkammerlinse ist, die Flanscheinrichtung (14) mit Bezug zum optischen Teil (12) in der Augenhinterkammer (30) nach vorne steht und das optische Teil (12) asymmetrisch bikonvex aufgebaut ist; dadurch gekennzeichnet, daß
die Linse (10) vollständig aus Hydrogel hergestellt ist und ihre Form im hydratisierten Zustand beibehält und
die vordere Fläche (16) des optischen Teils (12) einen größeren Krümmungsradius besitzt als die hintere Fläche (18) des optischen Teils (12).

2. Implantierbare Augenlinse (10) nach Anspruch 1, wobei die Flanscheinrichtung (14) mit Bezug zum optischen Teil (12) nach vorne steht.

3. Implantierbare Augenlinse (10) nach Anspruch 1, wobei die Flanscheinrichtung (14) ein Paar Flansche besitzt, die aus den gegenüberliegenden Seiten des optischen Teils (12) seitlich vorstehen.

4. Implantierbare Augenlinse (10) nach Anspruch 3, wobei die Flansche nicht perforiert sind.

## Revendications

1. Lentille intraoculaire autoportante (10) convenant pour l'implantation dans la chambre postérieure (30) de l'oeil humain en remplacement de la lentille naturelle du cristallin et comprenant :
une partie optique (12) ayant une surface antérieure (18) et une surface postérieure (16), ladite partie optique (12) étant suffisamment épaisse et rigide pour assurer une correction optique stable ; et
des moyens de bride (14) ayant une surface antérieure et une surface postérieure, lesdits moyens de bride (14) étant flexibles et servant à supporter et retenir la lentille (10) en place dans l'oeil après l'implantation, sans fixation à l'iris de l'oeil ;
ladite lentille (10) étant une lentille de chambre postérieure, lesdits moyens de brides (14) s'étendant vers l'avant par rapport à la partie optique (12) dans la chambre postérieure (30) de l'oeil et la partie optique (12) étant de construction asymétrique biconvexe ;
caractérisée en ce que ladite lentille (10) est entièrement réalisée en hydrogel et conserve sa forme à l'état hydraté et en ce que la surface postérieure (16) de la partie optique (12) a un rayon de courbure plus grand que celui de la surface antérieure (18) de la partie optique (12).

2. Lentille intraoculaire (10) selon la revendication 1, caractérisée en ce que lesdits moyens de bride (14) s'étendent vers l'avant par rapport à la partie optique (12).

3. Lentille intraoculaire (10) selon la revendication 1, caractérisée en ce que lesdits moyens de bride (14) comportent une paire de brides s'étendant latéralement à partir des côtés opposés de la partie optique (12).

4. Lentille intraoculaire (10) selon la revendication 3, caractérisée en ce que les brides ne sont pas perforées.
